# EUROPEAN PATENT SPECIFICATION

(11) **EP 1 291 034 B1**
(45) Date of publication and mention of the grant of the patent: **31.05.2006**
(21) Application number: 01307682.3
(22) Date of filing: 10.09.2001
(51) Int. Cl.: A61M 25/01

(54) **A catheter positioning device**
Anordnung zum Positionieren von Kathetern
Dispositif de positionnement de cathéter

(43) Date of publication of application: 12.03.2003
(73) Proprietor: Thermocore Medical Systems N.V., 9820 Merelbeke, Gent (BE)
(72) Inventor: Williams, John Vaughan, Cambridge CB4 8TL (GB); Diamantopoulos, Leonidas, 3140 Keerbergen (BE)
(74) Representative: Finnie, Peter John

(56) References cited:
- EP-A- 0 321 796
- WO-A-01/74263
- WO-A-99/45994
- US-A- 5 350 101
- US-A- 5 690 645
- US-A- 5 876 375
- US-A- 5 941 871
- US-A- 5 997 462

## Description

### Field of the Invention

The present invention relates to medical devices for controlling the positioning of an intra vascular catheter device. In particular, the present invention is concemed with a positioning device for relative positioning of lumen in a multi-lumen intravascular catheter.

### Background to the Invention

The human vascular system may suffer from a number of problems. These may broadly be characterised as cardiovascular and peripheral vascular disease. Among the types of disease, atherosclerosis is a particular problem. Atherosclerotic plaque can develop in a patient's cardiovascular system. The plaque can be quite extensive and occlude a substantial length of the vessel. Additionally, the plaque may be inflamed and unstable, such plaque being subject to rupture, erosion or ulceration which can cause the patient to experience a myocardial infarction, thrombosis or other traumatic and unwanted effects.

Our co-pending International Application No. PCT/EP01/04401 discloses a vascular catheter apparatus for temperature measurement of vascular tissue. Importantly, it has been reported that unstable and inflamed plaque can cause the temperature of the artery wall to elevate up to 2.5°C proximate the inflamed plaque. With the vascular catheter apparatus described in PCT/EP01/04401, detection of the temperature at the vascular wall is enabled. The temperature information is subsequently transferred via the carrier to a remote device where the wall temperature can be detected and recorded. The device is able to locate inflamed plaque by monitoring the vascular wall for elevated temperatures. This may be achieved by measuring temperature relative to normal segments of a vessel or absolute temperature values.

The thermography catheter is inserted into the target tissue using usual catheterisation techniques. This usually involves the use of a guide catheter.

Problems associated with systems incorporating a guide catheter include its relative positioning with respect to the catheter. For example, where it is desired to deliver a catheter to the coronary arteries, the guide catheter is maneuvered to the opening of the coronary arteries by a physician, in order that the catheter may be introduced to the coronary arteries. A problem may arise when the catheter is moved in the coronary arteries. As the physician tries to maneuver the catheter in the cardiac tissue, this may cause the guide catheter to be pushed away from the entrance to the coronary artery, consequently causing the catheter to be dragged out of the coronary artery. This problem is exaggerated where multi-lumen catheters are employed, such as the thermography catheter described in PCT/EP01/04401.

In order to accurately identify regions of unstable plaque, the vascular catheter apparatus described in PCT/EP01/04401 is controlled by a positioning device, referred to as a pull-back device. The pull-back device includes a number of lumen mounts that can be selectively connectable to a drive mechanism that can be used to manipulate and maneuver a catheter within a guide catheter and cardiac tissue. A guide catheter mount is fixed relative to the patient so that the guide catheter, once in position, does not move relative to the patient.

A problem associated with such a system is that the length of guide catheter remaining outside the patient is dependent on the size of the patient. For example, interventional cardiovascular treatment of a large patient will leave a shorter length of guide catheter and catheter outside the patient than treatment of the equivalent region in a smaller patient. Furthermore, the relative length of catheter to guide catheter left outside the patient for treatment of different regions, eg. peripheral heart tissue rather than the main coronary arteries, will differ. This poses a problem as, in use, the guide catheter and catheter must both be fixed to the positioning device in what is a fixed relative separation dictated by the physical dimensions of the positioning device and the lumen mounts.

### Summary of the Invention

According to a first aspect of the invention, a guide catheter extension piece comprises a substantially rigid tubular section fixed to a flexible tubular section, the rigid section being adapted for sealing engagement within a compression fitting of a Y-piece haemostasis valve, and a sealing element within the extension piece for engaging, in use, a catheter guided within the extension piece.

Where positioning of the catheter, therefore translational movement within the vascular tissue (therefore also within the guide catheter and guide catheter extension) is required, the arrangement allows the junction between the guide catheter extension and guide catheter to be sealed by tightening the compression fitting, but does not allow the junction to impinge on the catheter within. The seal is preferably achieved by providing a sealing element in the guide catheter extension which forms a low friction, slidable seal with the sheath of the catheter. Thus the catheter is able to be moved and positioned within the apparatus without undue friction being applied to the catheter. This is particularly important as a Y-piece, in addition to being used as the injection point for contrast medium into the patient, is also used as a pressure measurement point during the interventional procedure. In order for the pressure of the patient to be reliably measured, the system must be substantially closed, otherwise the pressure will vent at a non closed section. This will lead to loss of pressure, loss of blood, and unreliable pressure readings. However, the present system maintains the pressure of the system as the guide catheter and guide catheter extension junction is sealed and the diameter of the catheter is generally slightly less than the diameter of the internal lumen of the guide catheter extension. Alternatively, the pressure is maintained by providing the above mentioned sealing element in the guide catheter which forms a low friction, slidable seal with the sheath of the catheter.

The distal end of the guide catheter extension is adapted for engagement with a standard Y-piece used in interventional cardiology, having a compression fitting. This substantially prevents loss of blood or fluid at the junction between the guide catheter and the guide catheter extension.

The guide catheter extension piece of the present invention is adapted to receive a catheter used in interventional cardiology. Preferably, the body of the guide catheter extension piece is substantially cylindrical in cross section and has a diameter in the range of 1-15 mm. Preferably the diameter is in the range of 2-10 mm, more preferably 3-7 mm. Preferably, the length of the guide catheter extension piece is in the range of 0.1 m to 1 m. More preferably, the length of the guide catheter extension piece is 0.15-0.5 m.

The body of the guide catheter extension piece may be formed from standard guide catheter materials. For example nylon, PTFE, polyurethane, polyethylene and nitinol and mixtures thereof may be used. It may also be made from metals such as aluminium, steel and alloys thereof.

The guide catheter extension piece preferably has a number of engagement points along its length. Notches, annular indentations, and any other suitable means may be used. Preferably there are 2-200 fixation points, more preferably 5-100, most preferably 10-50 fixation points. These engagement means enable the guide catheter extension piece to be fixed in place, at selected positions over its length, on a catheter positioning device.

The guide catheter extension piece comprises a distal and a proximal end. Preferably, the distal end is adapted for engagement with the guide catheter, while the proximal end is adapted for engagement with the catheter positioning device.

In a most preferred embodiment, the distal end of the guide catheter extension piece comprises a substantially rigid tubular section which is fixed to a flexible section, and which is co-axial therewith. The rigid tubular section may be integrally moulded with the flexible section. Alternatively, it is fixed to the flexible section by any suitable means, for example, glue, soldering, welding and the like.

According to a second aspect of the present invention, a vascular catheter positioning system comprises: a vascular catheter, a guide catheter fitted to a Y-piece haemostasis valve having a compression fitting; a catheter positioning device; and, a guide catheter extension piece comprising a substantially rigid tubular section fixed to a flexible tubular section, the rigid section being adapted for sealing engagement within the compression fitting, and a sealing element within the extension piece for engaging, in use, a catheter guided within the extension piece, wherein the catheter positioning device is adapted to engage the proximal end of the vascular catheter and also engage the flexible tubular section of the guide catheter extension so that the vascular catheter can subsequently be manipulated relative to the guide catheter without movement of the guide catheter.

The present invention allows the distance between a guide catheter and a positioning device to be manipulated by the user. Thus the guide catheter may be fixed in position relative to both patient and positioning device, while providing the optimum distance between the effective length of the guide catheter (guide catheter and guide catheter extension piece) and the points at which the catheter is fixed to the positioning device.

The catheter positioning device is preferably a type for positioning a catheter and comprises a first lumen mount for holding a first lumen of the catheter, a second lumen mount for holding the guide catheter extension piece, and a drive mechanism, wherein the first lumen mount is selectively connectable to the drive mechanism for relative movement with respect to the second lumen mount.

The second lumen mount preferably includes a bracket, preferably adapted for engagement with the guide catheter extension. The bracket is usually located at one end of an extension arm, while the other end is connected to the body of the positioning device.

In a preferred embodiment, the positioning device is a pull-back device which is used for positioning and/or controlled withdrawal of a catheter.

In a particularly preferred embodiment, the present invention is used in concert with a vascular catheter apparatus, especially multi-lumen and/or sheathed catheters, which require precise positioning and or maneuvering within vascular tissue. For example, the present invention finds particular utility with catheters used for measurement of a physical parameter and/or treatment of vascular tissue, and which comprise a flexible body, and at least one sensor and/or treatment means.

Treatment means include those usually used in interventional cardiology, such as ablation apparatus (for example electrodes), drug delivery ports, tissue removal apparatus, stents and stent positioning means (for example, balloons or sleeves) and the like.

In a particularly preferred embodiment, the present invention is used in concert with a vascular catheter apparatus, preferably a catheter apparatus comprising a body, at least one resiliently biased projection depended from the body, a sensor carried by the projection, and an electrical carrier connected to the sensor for transmitting data from the sensor to a remote device, wherein the electrical carrier is coiled. Such a device is described in our earlier filed European patent application no. 01306599.0 In this embodiment, the electrical connection is coiled to reduce the strain at critical points where it is necessary to maintain a seal, and hence electrical isolation. The design is also especially suitable for use with a vascular thermography catheter apparatus of the type described in our earlier filed International patent application no. PCT/EP01/04401.

### Brief Description of the Drawings

Examples of the present invention will now be described in detail with reference to the accompanying drawings, in which:
Figure 1 shows a schematic diagram of a system for conducting vascular catheterisation of a patient;
Figure 2 shows a side view of the distal tip of a thermography catheter device in a temperature sensing (deployed) configuration;
Figure 3 shows a side view of the distal tip of the device in a non-temperature sensing (retracted) configuration;
Figure 4 shows the pull-back device in side view;
Figure 5 shows the pull-back device in plan view;
Figure 6 shows a section of the guide catheter extension distal end; and,
Figure 7 is a flow diagram illustrating the steps involved with conducting intravascular catheterisation of a patient and the associated data capture and image processing.

### Detailed Description

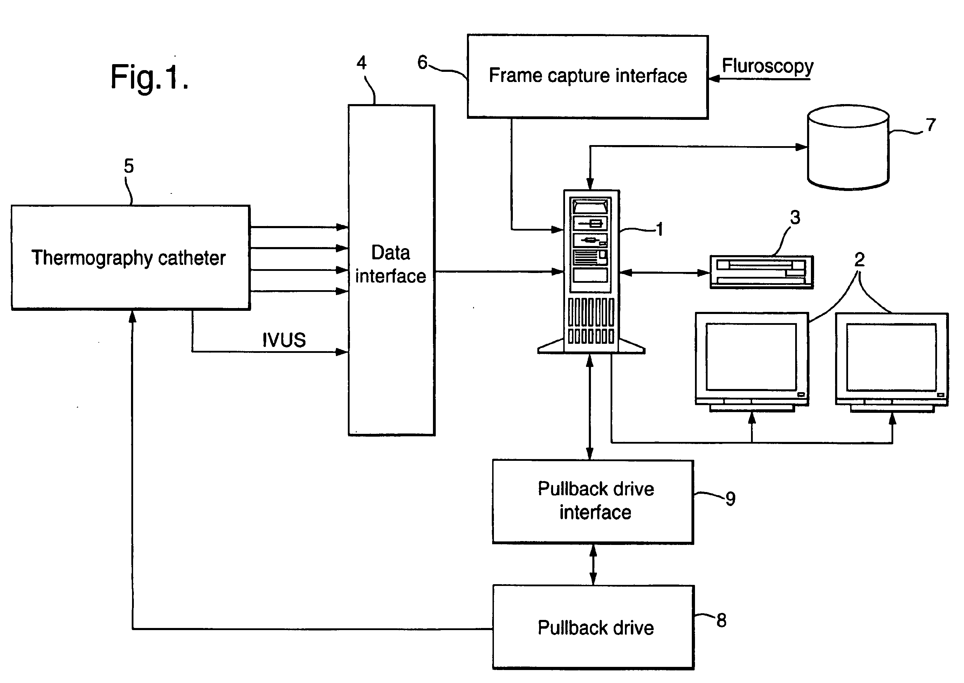

Figure 1 is a schematic diagram of a system for conducting vascular catheterisation of a patient.

The system includes a personal computer (PC) 1 that presents a graphical user interface (GUI) via a number of monitors 2. The user interface system is based on a Microsoft Windows^{™} platform. Multiple windows may be used to acquire/project data from/to the user. Although not shown, the PC can accept user inputs via a keyboard and mouse, or other pointing device, in the usual manner. The PC includes a number of data stores 7, which may be external, and a CD ROM reader/writer device 3.

The PC is coupled via a data interface 4 to a thermography catheter 5, details of which will be described below. In this example, the thermography catheter 5 transmits four channels (one for each sensor) which are received by the data interface 4. An analogue temperature data signal on each channel is converted to a digital signal using an A/D converter within the data interface 4 at a user configured sampling rate of up to 2.5 KHz. Typically, the sampling rate would be set at around 25 to 50 Hz to reduce the quantity of data acquired.

The data interface 4 includes a multiplexer (not shown) that combines the four digital channels into a single time division multiplexed (TDM) signal. This TDM signal is coupled to the PC over a PCI bus. The data from each channel are written into an area of memory within the data store 7 reserved for that channel where they can subsequently be retrieved for data processing along with the corresponding time sequenced data from other channels and image data from other sources.

The temperature data from the thermography catheter 5 are introduced to the system software running on the PC using function calls. Temperature data are input to the software as the actual voltage at the A/D hardware inputs, and therefore they have to be converted to temperature. A sensor data convert function handles this process.

The system is designed to be used in conjunction with a fluoroscopy x-ray apparatus and therefore includes a video frame capture interface 6 that couples fluoroscopy video data inputs to the PC via a PCI bus. Similarly, it can be used in conjunction with intravascular ultra-sound (IVUS) image data fed from the thermography catheter 5 (when provided with the appropriate hardware). The system software allocates sufficient memory area to the systems memory for this data, taking into account the current system configuration, for example sampling rate, recording time, and video frame size. A memory handle hDib is used to map video data directly through the PCI bus from the video frame capture interface 6 to this allocated area in memory. hDib memory is divided into i equal chunks, each of a size equal to the frame capture interface frame-buffer. Optionally, hDib [i] data can also be mapped to a memory area of a screen-video buffer, giving capability of live preview during recording. Each time the software records an x group of four (or more) temperature measurements, it prompts for a frame capture at hDib [x]. A user configuration file determines the ratio between temperature data:fluoroscopy video frame capture.

Whilst in normal circumstances the thermography catheter 5 is inserted manually, it is intended that when performing vascular measurements the thermography catheter 5 is pulled back relative to a predetermined start position using an electro-mechanical pull-back drive 8 coupled to the body of the catheter. The pull-back drive 8 is controlled by the PC via a pull-back drive interface 9. The system software accesses user-defined configuration files to get the necessary information about controlling the systems automatic pull-back interface 9. Data sampling rate, recording duration and pre-selected retraction rate are taken into consideration for adjusting the pull-back speed. The software routines control a D/A converter (not shown) that feeds the input of the pull-back interface 9 with an appropriate control voltage. The controlled pull-back process will be described in more detail below.

Temperature data plotting may be both on-line and/or off-line. In an on-line mode, the monitor presents a temperature/time-distance graph, where temperature is continuously plotted as connected dots. In an off-line mode, temperature data can be loaded from the data store 7 (or other media) and plotted on the screen graph. The user can scroll to different time/temperature locations, while several automated functions may be provided, for example auto min-max marking, colour coding of temperature on a bullseye graph, colour thermal maps, and 3D temperature coding on a cylinder model. In the latter case, an artificial colour 3D cylinder that represents the vessel is divided into splines equal to the temperature channels. The channel temperature is coded on each spline with colours varying from dark-blue (minimum temperature) to flashing-red (maximum temperature). The user can rotate the cylinder as he wishes in a virtual 3D world. The focus is set to the specific time/distance that corresponds to the mouse position on the screen temperature/time graph. 3D position control is performed using multi cubic-bezier lines, where the curvation control points change in relation to the cylinders position in the virtual world. A separate window shows numeric details for the particular time/distance position. Video frame data from simultaneous fluoroscopy/IVUS are plotted as image frames in a separate window. By moving to a specific time/temperature position, the corresponding video frame is automatically projected. In this way, temperature and video frames are accurately synchronised.

The system software is designed to provide basic and advanced image processing functions for the captured fluoroscopy/IVUS video frames, such as filtering and on-screen measurement functions. The user can filter the captured frame to discard unwanted information while focusing on the desired one. There are several auto-filter options as well as manual adjustment of the image curve. In addition, the user can calibrate the system and proceed in performing on-screen measurements of both distances and/or areas. Automatic routines perform quantification of the measurements giving significant information on lesion characteristics. The temperature can also be colour coded on the fluoroscopy frame, providing unique information about the correlation between temperature and morphology.

By using temperature data and video frame data, the system software uses advanced algorithms based on interpolation and fractal theory to plot a 3D reconstruction of the vessel under measurement with colour coding of temperature. The user can freely move the virtual camera inside the reconstructed vessel in 360°, and/or fly-through the vessel. 2D reconstructions are also provided. Temperature data can be processed on the basis of mean temperature, or on a channel-by-channel basis.

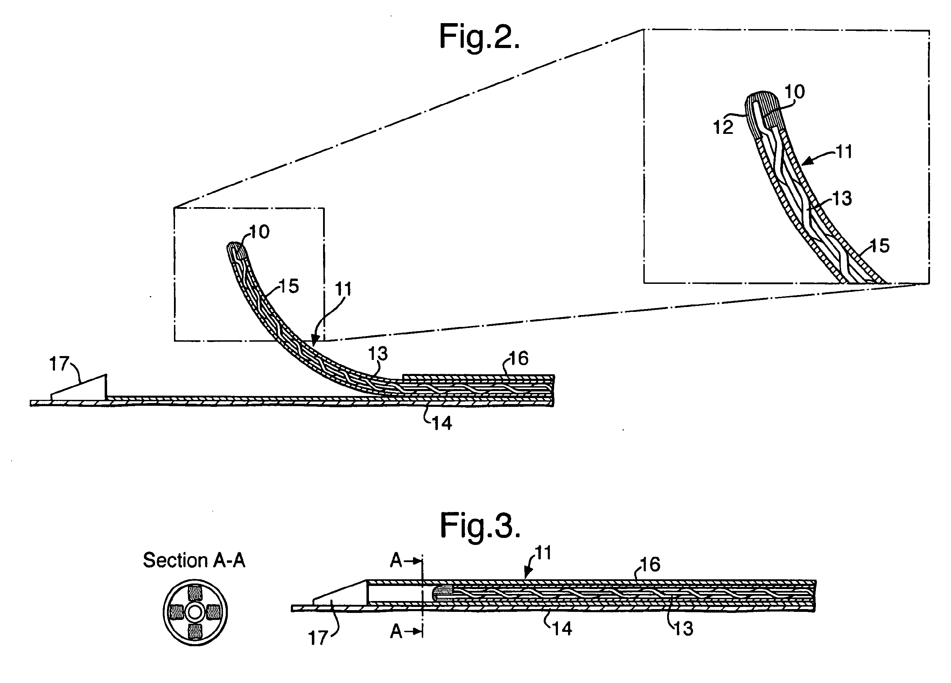

Figures 2 and 3 show an example of the distal tip of a thermography catheter incorporating sensors 10 mounted circumferentially about a central lumen 14. In this example, four sensors 10 are mounted on resiliently biased projections 11 circumferentially about the central lumen at 90° intervals, although only one sensor is shown here for the sake of clarity. The projections 11 are made of NiTinol.

The sensors 10 are NTC thermistors. Such thermistors prove extremely reliable regarding the relation between the temperature changes and resistance changes. An NTC thermistor having a 30 KΩ impedance at 25°C typically maintains linearity between 35°C and 45°C, at a resolution of 0.01°C - 0.1°C.

The construction of the thermistors 10 are that of two rectangular plates with a semi-conductor material in the centre. The thermistor has dimensions in the range of 0.25mm - 5mm, and a caliper less than 1 mm.

Each thermistor 10 is attached to the end of each projection 11 by bonding with an thermally conducting epoxy glue 12. Each thermistor 10 is connected to an insulated bifilar wire 13. The wire 13 has a low impedence and is constructed from nickel and/or copper. This wire provides an electrical connection with the proximal end of the device (not shown).

As shown in the Figures, the wire 13 is coiled around the length of the projection 11. This feature has the effect of substantially eliminating strain when the projection 11 flexes. The pitch of the coil is typically arranged to be such that there are 5 to 10 turns over a length of 10 mm. As will be described below, a heat shrink wrapping 15 is applied over the projection 11 to prevent damage to the wire 13 during retraction and replacement of an outer sheath 16. The heat shrink wrapping also provides an additional degree of electrical isolation.

To assemble a projection, a NiTinol arm is first pretreated by placing it in a bending tool and heating to around 700°C to impart a bend in the arm. The NiTinol arm is then held straight in a chuck and a thermistor /bifilar wire assembly is attached to a free end of the arm using a UV cure adhesive. The wire 13 is then spun around the length of the NiTinol arm. Finally, the heat shrink wrapping 15 is placed over the length of the NiTinol arm to a point just beyond that of the thermistor. In this example , the heat shrink wrapping is supplied as a polyester tube that is cut to length. An epoxy resin is then injected into the end of the tube. The assembly is subsequently heat treated to shrink the tube and set the epoxy resin. The heat shrink wrapping is then trimmed back to expose at least part of the epoxy resin coated thermistor, while maintaining electrical isolation of the bifilar wires. After heat treatment, the heat shrink has a wall thickness of around 10µm.

As shown in the Figures , the thermography catheter is mounted on an angioplasty guide wire (not shown) which runs through the central lumen 14 and a guide member 17 which defines the tip of the thermography catheter.

In use, the apparatus may be actuated between a non-wall-temperature sensing configuration and a temperature sensing configuration. The non-temperature sensing configuration is hereinafter referred to as the retracted configuration. The temperature sensing configuration is hereinafter referred to as the deployed configuration. An example of the deployed configuration is shown in Figure 2. An example of the retracted configuration is shown in Figure 3.

In the retracted configuration, the sheath 16 encompasses the projections 11 so that they are constrained to lie parallel to the longitudinal axis of the catheter and therefore cannot take up a deployed position. The sheath 16 extends as far as the rear end of the guide member 17 and may or may not overlap the guide member. Where there is overlap, a smooth profile of the catheter is maintained. Any protrusions from the thermography catheter which could lead to damage of the vascular wall, are minimised in the retracted configuration. This is particularly important where a vessel is angulated or there is bifurcation of the vessel. Such features lead to bending of the thermography catheter and would emphasise any protrusions. Hence, in this example the sheath 16 and the guide member 17 present a smooth profile when adjacent to one another in the retracted configuration.

To adopt the deployed configuration, the sheath 16 is withdrawn away from the extreme distal tip i.e., away from the guide member 17, towards the proximal section, to expose the projections 11. When the sheath 16 is withdrawn to the extent shown in Figure 2, the resiliently biased projections 11 take up the deployed configuration. It should be noted that the sheath is controlled from the proximal end of the apparatus and is not shown in its entirety in the Figures.

The projections 11 individually extend a certain distance (r) away from the longitudinal axis of the catheter. In the deployed configuration, r has a value in the range of 2-4 mm. However, r is not fixed and varies with the diameter of the vascular tissue being measured due to the flexibility of the projections 11.

Different diameter catheters may be used for different diameters of vascular tissue. However, as it is desirable to minimize the diameter of catheters in all interventional vascular treatments, it is desirable to adapt the length of the projections and/or the angle to which the projections may extend away from the central lumen depending on the dimensions of the vascular tissue being measured rather than increasing catheter body dimensions. Thus, the projections for a large blood vessel, for example 8 mm diameter, will generally require a length of projection in the range of 5 mm to 10 mm. Smaller diameter vascular tissue, for example 2.5 mm diameter, will generally require a length of projection in the range of 2 mm to 6 mm. Typically, the ratio of the area of the cross-sectional profiles of the apparatus in the deployed to retracted configurations is up to 4:1.

The thermography catheter includes a valve system (not shown) allowing the annular gap between the sheath and the intermediate lumen to be flushed in an adequate way, thus minimising the possibility of air bubbles or debris within the sheath. Such a valve is constructed to enable engagement by a 2 mm, 5 mm, or 10 mm, 6° luer syringe. The thermography catheter may be flushed with a suitable fluid such as saline. When flushing the catheter, fluid should exit via the distal tip of the catheter, indicating proper flushing of the sheath. In addition, the catheter includes a female luer fitting (not shown) attached to the proximal end of the central lumen, to enable the central lumen to be flushed in a similar way to the sheath.

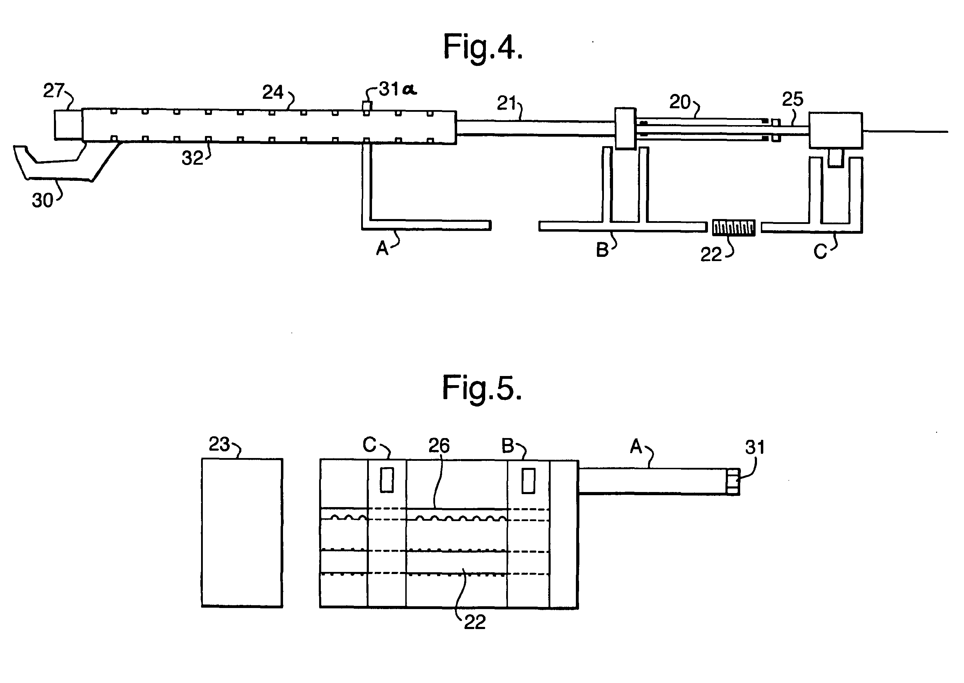

The body of a pull-back device is illustrated in Figures 4 and 5. The proximal section of the thermography catheter described above is constructed to enable remote deployment and retraction of the projections. This is effected via manipulation of the sheath. A two-lumen telescopic construction 20 is used to manipulate the sheath 21 between the retracted and the deployed configuration. One lumen is connected to, or integral with, the outer sheath and can slide over an adjacent lumen which comprises or is connected to one of the lumen housed within the sheath. Rotation of one tube inside the other is prevented by slotting of the lumen or other features on the lumen. Additionally, scaling markings (not shown), may be provided to avoid over-retraction of the tubes.

The pull-back device includes a drive module 23 which includes a motor, gearing system, typically a speed reducer, control and monitoring means, and engagement gear for a driving rod 22. The drive module may be formed separately from the body of the pull-back device so that it may be reused. The body of the pull-back device must be kept sterile and may be formed from a material such as polyurethane. This allows the body to be cheaply and easily produced and may be disposable. Alternatively, or additionally, the pull-back device may be enclosed in a sterile, flexible plastic sheath when in use, so as to maintain sterility.

The pull-back device comprises a driving rod 22, adapted for engagement with an engagement gear of the drive module 23 and mount C. Mounts C and B are adapted to engage the central/intermediate lumen 25 and the sheath lumen 21 respectively. A Mount A is provided which is adapted to engage the guide catheter extension 24. Mount A includes a bracket 31a for connection of mount A to the guide catheter extension fixation points 32. When engaged, mount B may be moved towards C to place the thermograph catheter in the open configuration. C may be selectively driven reversibly over a range of travel (usually about 60 mm) suitable for withdrawal of the catheter apparatus over the measured region. The driving rod 22 is a worm-screw type which interacts with the engagement gear of the drive module 23, thus providing a smoothly driven apparatus.

The mounts B and C may individually be locked in position relative to one another or may be selectively unlocked in order to allow movement of the lumen 25, sheath 21 and guide catheter 24 relative to one another.

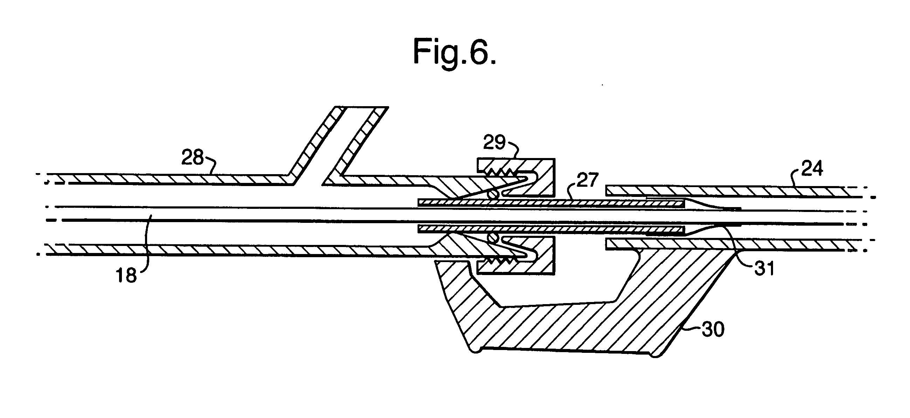

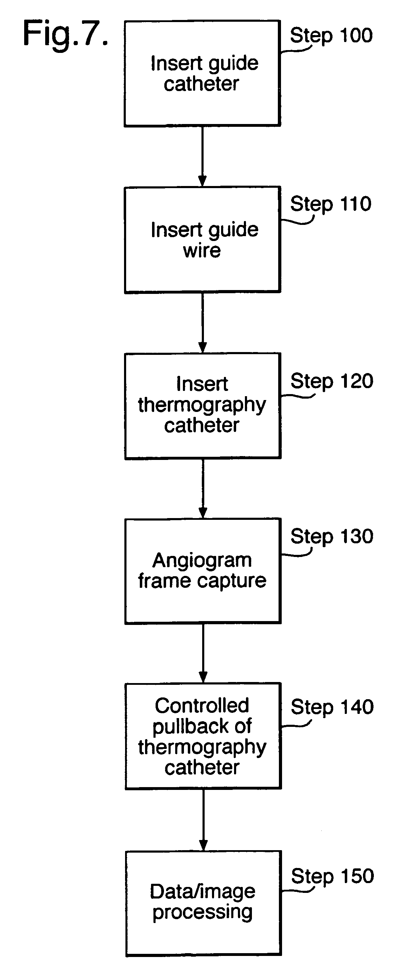

With reference to Figures 6 and 7, in use, the sequence of events begins with the insertion of a guiding catheter into the area of general interest (step 100), for example the cardiac region. Where, for example, the coronary arteries are to be examined, the guiding catheter is inserted so that it is adjacent the opening of the coronary arteries (step 110). An angioplasty guide wire is then inserted into the coronary artery, past the point of specific interest. The guide wire is usually inserted with the aid of standard fluoroscopic techniques, as is the guide catheter.

The guide catheter, when in place over the entrance to the coronary (or other target) artery will protrude a distance from the patient once in place. This is then fixed to the guide catheter extension 24. The guide catheter extension will be fixed to the guide catheter by inserting the non-compressible tube 27 into the Y-piece 28. The gland nut 29 and o-ring seal (compression fitting) is tightened to seal the joint between the guide catheter and guide catheter extension and a securing means 30 is provided which holds the Y-piece in place relative to the guide catheter extension. Alternatively, the outside surface of the non-compressible tube may be profiled with shallow circumferential grooves, to ensure that the tube will not pull out when held in the compression fitting of the Y-piece (not shown).

A seal element 31 is provided within the guide catheter extension. This is sandwiched between the non-compressible tube and the guide catheter extension body. This provides a sealing engagement between the nobn-compressible tube and the catheter.

Once the guide catheter, guide catheter extension and guide wire are in position, the catheter of the present invention is maneuvered over the guide wire to a position beyond the specific area of interest in the coronary artery (step 120) with the aid of fluoroscopy. The catheter is then fixed in position on the pull-back device by clipping into mounts B and C. The guide catheter extension is then fixed in position on the mount A, at a fixation point along its length which optimises the distance between mount A and B and C. Thus, the guide catheter extension should be fixed to mount A so that the catheter may be mounted on mounts B and C in a closed configuration.

An angiogram is taken (step 130) to assess the position of the catheter in the vascular tissue. This image is saved and the position of the catheter is marked on the image so as to define a starting point for the controlled pull-back step.

The sheath 21 is then be retracted to allow the projections to adopt the deployed configuration. This is achieved by moving mount B towards mount C (usually manually). Mount C at this time is locked relative to mount A. Once the sheath 21 is retracted sufficiently to allow expansion of the resiliently biased projections, mount B is locked in position and mount C is pulled back by the drive mechanism until the projections are housed in the sheath. This is feasible if the sheath 21 is retracted sufficiently (equal or greater than the length of the pull-back distance during which measurement takes place) to allow the intermediate/central lumen 25 to be retracted in the sheath 21 without the sheath impacting on the projections along the length of measurement.

Alternatively, the mount B and C are locked in position once the thermography catheter is in the deployed configuration and both mounts are pulled back by the drive mechanism.

The locking mechanism includes a stopper rod 26. This is provided with graduations capable of engaging electrically actuated locking pins (not shown) within mounts B and/or C. A similar set of electrically actuated locking pins (not shown) within the same mounts are used to selectively connect the mounts to the drive rod 22. A set of locking pins on any particular mount may not be connected to both the drive rod 22 and the stopper rod 26 simultaneously. Thus, each mount is either in drive or stop mode. Alternatively a ratchet mechanism may be provided as the locking mechanism.

When the mount C is in drive mode, it moves relative to mount A and B. Mount C cannot be moved towards mount B when attached to the pull-back device.

The catheter may be marked to indicate when the sensors are in a deployed or in a retracted position. This may be achieved by provision of a telescopic tubing 20 with appropriate indicators or by simply marking the extreme deployed or retracted position on the apparatus.

Controlled pull-back of the catheter then takes place (step 140). The pull-back takes place at a constant speed and is controllable by the user. Pull-back typically takes place at speeds of 0.1 to 2 mm in divisions of 0.1 mm or so.

The pull-back takes place over a distance of the vascular tissue being measured. Temperature readings may be taken intermittently or substantially continuously. The data transmitted by the sensors from the vascular wall is captured for data and image processing (step 150) together with a fluoroscopy/IVUS image frame.

As the catheter is withdrawn inside the artery, the projections automatically adjust their angle following the wall's morphology without losing the desired thermal contact. The result is that the thermal contact between the sensors and the wall is continuously maintained, even when the catheter is crossing very irregular plaque formations.

Once the pull-back has been completed, the central/intermediate lumens are retracted such that the projections are withdrawn into the sheath 21 in order to place the sensors in the retracted configuration. This restores the original smooth profile of the catheter. The catheter may then be detached from the pull-back device and withdrawn from the patient or may be reinserted into the same or another blood vessel in order to take another reading. Alternatively, the catheter may be reinserted in order to enable a therapeutic or surgical intervention.

## Claims

1. A guide catheter extension piece (24) comprising a substantially rigid tubular section (27) fixed to a flexible tubular section, the rigid section (27) being adapted for sealing engagement within a compression fitting of a Y-piece haemostasis valve, and a sealing element (31) within the extension piece (24) for engaging, in use, a catheter guided within the extension piece.

2. A guide catheter according to claim 1, wherein the diameter of the guide catheter extension piece is in the range of 1 mm - 15 mm, preferably 2 mm - 10 mm, more preferably 3 mm - 7 mm.

3. A guide catheter extension piece according to claim 1 or claim 2, wherein the length of the guide catheter extension piece is in the range of 0.1 m to 1 m, preferably 0.15 m to 0.5 m.

4. A guide catheter extension piece according to any preceding claim, in which the guide catheter extension piece has a number of engagement points (32) along its length.

5. A combination of a vascular catheter and a guide catheter extension piece according to any preceding claim.

6. A vascular catheter positioning system, comprising:
a vascular catheter (25);
a guide catheter (18) fitted to a Y-piece haemostasis valve (28) having a compression fitting (29);
a catheter positioning device (22, A, C) and,
a guide catheter extension piece (24) comprising a substantially rigid tubular section (27) fixed to a flexible tubular section, the rigid section (27) being adapted for sealing engagement within the compression fitting (29), and a sealing element (31) within the extension piece for engaging, in use, a catheter guided within the extension piece,
wherein the catheter positioning device (22, A, C) is adapted to engage the proximal end of the vascular catheter (25) and also engage the flexible tubular section of the guide catheter extension (24) so that the vascular catheter can subsequently be manipulated relative to the guide catheter without movement of the guide catheter.

7. A system according to claim 6, in which the catheter positioning device comprises a first lumen mount (C) for holding a first lumen (25) of the vascular catheter, a second lumen mount (A) for holding the guide catheter extension piece (24), and a drive mechanism (22), wherein the first lumen mount is selectively connectable to the drive mechanism for relative movement with respect to the second lumen mount.

8. A system according to claim 6 or claim 7, in which the guide catheter extension piece has a number of points (32) adapted for engagement with the catheter positioning device.

9. A system according to any of claims 6 to 8, wherein the length of the guide catheter extension piece is in the range of 0.1 m to 1 m, preferably 0.15 m to 0.5 m.

## Patentansprüche

1. Führungskatheterverlängerungsstück (24) umfassend einen im wesentlichen festen rohrförmigen Abschnitt (27), der an einem biegbaren rohrförmigen Abschnitt befestigt ist, wobei der feste Abschnitt (27) ausgelegt ist für einen dichtenden Eingriff innerhalb eines Druck-Fittings eines Hämostaseventils mit Y-Stück, sowie ein Dichtelement (31) in dem Verlängerungsstück (24), um im Gebrauch einen in dem Verlängerungsstück geführten Katheter zu greifen.

2. Führungskatheter nach Anspruch 1, bei welchem der Durchmesser des Führungskatheterverlängerungsstücks im Bereich von 1 mm - 15 mm, vorzugsweise 2 mm - 10 mm, besonders bevorzugt 3 mm - 7 mm, liegt.

3. Führungskatheterverlängerungsstück nach Anspruch 1 oder Anspruch 2, bei welchem die Länge des Führungskatheterverlängerungsstücks im Bereich von 0,1 m bis 1 m, vorzugsweise 0,15 m bis 0,5 m, liegt.

4. Führungskatheterverlängerungsstück nach einem der vorstehenden Ansprüche, bei welchem das Führungskatheterverlängerungsstück mehrere Eingriffsstellen (32) entlang seiner Länge hat.

5. Kombination eines vaskulären Katheters und eines Führungskatheterverlängerungsstück gemäß einem der vorstehenden Ansprüche.

6. Positioniersystem für einen vaskulären Katheter, umfassend:
einen vaskulären Katheter (25);
einen Führungskatheter (18), der an einem Hämostaseventil (28) mit Y-Stück angebracht ist, das ein Druck-Fitting (29) hat;
eine Katheterpositioniervorrichtung (22, A, C); und
ein Führungskatheterverlängerungsstück (24), umfassend einen im wesentlichen festen rohrförmigen Abschnitt (27), der an einem biegbaren rohrförmigen Abschnitt befestigt ist, wobei der feste Abschnitt (27) ausgelegt ist zum dichtenden Eingriff innerhalb des Druck-Fittings (29) und
ein Dichtelement (31) innerhalb des Verlängerungsstücks, um im Gebrauch einen in dem Verlängerungsstück geführten Katheter zu greifen, wobei die Katheterpositioniervorrichtung (22, A, C) ausgelegt ist, um das proximale Ende des vaskulären Katheters (25) zu greifen und auch den biegbaren rohrförmigen Abschnitt der Führungskatheterverlängerung (24) zu greifen, so daß der vaskuläre Katheter darauffolgend relativ zu dem Führungskatheter ohne Bewegung des Führungskatheters bedienbar ist.

7. System nach Anspruch 6, bei dem die Katheterpositioniervorrichtung eine Befestigung (C) für ein erstes Lumen umfaßt, um ein erstes Lumen (25) des vaskulären Katheters zu halten, eine Befestigung (A) für ein zweites Lumen, um das Führungskatheterverlängerungsstück (24) zu halten und einen Antriebsmechanismus (22), wobei die Befestigung für das erste Lumen wahlweise mit dem Antriebsmechanismus verbindbar ist zum Zwecke der relativen Bewegung in Bezug auf die Befestigung für das zweite Lumen

8. System nach Anspruch 6 oder Anspruch 7, bei welchem das Führungskatheterverlängerungsstück mehrere Stellen (32) hat, die ausgelegt sind für einen Eingriff mit der Katheterpositioniervorrichtung.

9. System nach einem der Ansprüche 6 bis 8, bei welchem die Länge des Führungskatheterverlängerungsstücks im Bereich von 0,1 m bis 1 m, vorzugsweise 0,15 m bis 0,5 m, liegt.

## Revendications

1. Pièce d'extension d'un guide de cathéter (24) comprenant une section tubulaire sensiblement rigide (27), fixée à une section tubulaire flexible, la section rigide (27) étant adaptée pour un engagement de scellement dans un montage à compression d'une vanne hémostatique en Y, et un élément de scellement (31) dans la pièce d'extension (24) pour l'engagement, lors de l'utilisation, d'un cathéter guidé dans la pièce d'extension.

2. Guide de cathéter selon la revendication 1, dans lequel le diamètre de la pièce d'extension du guide de cathéter se situe dans l'intervalle allant de 1 mm à 15 mm, de préférence de 2 mm à 10 mm, de manière plus préférée de 3 mm à 7 mm.

3. Guide de cathéter selon la revendication 1 ou 2, dans lequel la longueur de la pièce d'extension du guide de cathéter se situe dans l'intervalle allant de 0,1 m à 1 m, de préférence de 0,15 m à 0,5 m.

4. Pièce d'extension du guide de cathéter selon l'une quelconque des revendications précédentes, dans laquelle la pièce d'extension du guide de cathéter présente un certain nombre de points d'engagement (32) sur sa longueur.

5. Combinaison d'un cathéter vasculaire et d'une pièce d'extension d'un guide de cathéter selon l'une quelconque des revendications précédentes.

6. Système de positionnement d'un cathéter vasculaire, comprenant :
un cathéter vasculaire (25) ;
un guide de cathéter (18), équipé d'une vanne hémostatique en Y (28), ayant un montage à compression (29) ;
un dispositif de positionnement du cathéter (22A, C), et
une pièce d'extension d'un guide de cathéter (24) comprenant une section tubulaire sensiblement rigide (27), fixée à une section tubulaire flexible, la section rigide (27) étant adaptée pour un engagement de scellement dans un montage à compression (29) et un élément de scellement (31) dans la pièce d'extension pour l'engagement, lors de l'utilisation, d'un cathéter guidé dans la pièce d'extension,
où le dispositif de positionnement du cathéter (22A, C) est adapté pour s'engager dans l'extrémité proximale du cathéter vasculaire (25) et également, s'engager dans la section tubulaire flexible de l'extension du guide de cathéter (24), de sorte que le cathéter vasculaire puisse ensuite être manipulé par rapport au guide de cathéter sans mouvement du guide de cathéter.

7. Système selon la revendication 6, dans lequel le dispositif de positionnement du cathéter comprend un premier montage de lumen (c) pour maintenir un premier lumen (25) du cathéter vasculaire et un mécanisme de direction (22), dans lequel le premier montage de lumen est connectable de manière sélective, pour diriger le mécanisme d'un mouvement relatif par rapport au deuxième montage de lumen.

8. Système selon la revendication 6 ou 7, dans lequel la pièce d'extension du guide de cathéter a un certain nombre de points (32) adaptés pour l'engagement avec le dispositif de positionnement du cathéter.

9. Système selon l'une quelconque des revendications 6 à 8, dans lequel la longueur de la pièce d'extension du guide de cathéter se situe dans l'intervalle allant de 0,1 m à 1 m, de préférence de 0,15 m à 0,5 m.
